# EUROPEAN PATENT SPECIFICATION

(11) **EP 3 706 844 B1**
(45) Date of publication and mention of the grant of the patent: **01.01.2025**
(21) Application number: 18876094.6
(22) Date of filing: 08.11.2018
(51) Int. Cl.: A61M 16/00, A61M 16/10, A61M 16/20

(54) **MEDICAL VENTILATOR SYSTEM AND METHOD FOR PROVIDING RESPIRATORY SUPPORT TO A PATIENT**
MEDIZINISCHES BEATMUNGSSYSTEM UND VERFAHREN ZUR BEATMUNGSUNTERSTÜTZUNG EINES PATIENTEN
SYSTÈME DE VENTILATEUR MÉDICAL ET PROCÉDÉ PERMETTANT DE FOURNIR UN SOUTIEN RESPIRATOIRE À UN PATIENT

(30) Priority: 08.11.2017 IN 201741039744
(43) Date of publication of application: 16.09.2020
(73) Proprietor: General Electric Company, Schenectady, NY 12345 (US)
(72) Inventor: CHAUDHRY, Gunaranjan, 560066 Bangalore Karnataka (IN); VIJAYARAGHAVAN, Sanjay, 560066 Bangalore Karnataka (IN); GIRIMAJI, Arun, 560066 Bangalore Karnataka (IN); SR, Prakash, 560066 Bangalore Karnataka (IN)
(74) Representative: Fennell, Gareth Charles
(86) International application number: PCT/US2018/059783
(87) International publication number: WO 2019/094561

(56) References cited:
- EP-A1- 0 796 119
- EP-A1- 2 106 819
- EP-B1- 0 796 119
- WO-A1-2013/096495
- DE-A1- 102004 011 907
- US-A1- 2008 196 720
- US-A1- 2008 196 720
- US-A1- 2011 000 489
- US-A1- 2015 083 135
- US-A1- 2016 271 359

## Description

### BACKGROUND

Embodiments of the present specification relate generally to medical ventilators, and more particularly to a ventilator system and method for controlling one or more parameters of gases that are used for providing respiratory support to a patient.

In general, medical ventilators are mechanical devices that are used to provide respiratory support to a patient. Typically, if a patient is weak or sedated, the patient may be unable to independently perform respiration functions. Hence, the medical ventilators are used for mechanical delivery of a breath to the patient. More specifically, the medical ventilators are used to deliver medical gas under a pressure that is sufficient to overcome patient's airway resistance to fill lungs in an inhalation phase. Further, in an exhalation phase, the medical ventilators may reduce the pressure of the medical gas, which in turn causes the delivered medical gas to flow out of the patient. These inhalation and exhalation phases are repeated alternately to mechanically deliver breath to the patient. In some examples, these medical ventilators may also be used to deliver anesthesia to the patient.

In conventional systems, the medical ventilators require a supply of pressurized medical gas to provide the respiratory support to the patient. In one system, pressurized supply tanks are used for supplying the medical gas to the medical ventilators. However, these supply tanks are also used for providing a drive gas that is used to actuate the medical ventilators. Hence, the supply tanks get used up frequently. Also, these pressurized supply tanks are expensive to use and cumbersome to transport. As a result, operating cost of the system may be substantially increased. In another system, a centralized unit is used for supplying the medical and drive gas. In particular, gas piping and connections are provided in each room in a hospital. Further, these gas connections are connected to the centralized unit to supply the pressurized medical gas to the medical ventilators. However, using the gas connections to supply the medical gas may restrict the mobility of the ventilators and may also increase the set-up cost and the maintenance cost of the system.

Thus, there is a need for an improved system and method for providing respiratory support to a patient.
DE 102004011907 discloses an artificial narcosis respiration unit which presents the drive gas for the dome, or for the dome and the control chamber of an overflow valve, with use of a double valve consisting of inhalation and exhalation valves. The unit further includes valves for switching the unit between narcosis and therapy modes of operation.
US2008196720 discloses a ventilatory system for providing ventilatory support to a patient without the need for an external source of pressurized drive gas. The ventilatory system comprises a drive pump and a controller such that the drive pump collects ambient air and may pressurize it to a pressure determined by the controller. The controller may signal to the drive pump to pressurize the collected ambient air to a first pressure for delivering ventilatory support to a patient and a second pressure for providing PEEP support to a patient. The controller may signal to the drive pump to deliver a targeted flow and/or volume of collected ambient air to the bellows to provide volumetric ventilatory support during inhalation and a PEEP support during exhalation.

### BRIEF DESCRIPTION

In accordance with aspects of the present specification, a ventilator system for providing respiratory support to a patient according to claim 1 is presented. The ventilator system includes a controller configured to generate a first control signal for a first time-period and a second control signal for a second time-period during an inspiration time of a ventilation cycle. Also, the ventilator system includes a rotary pump electrically coupled to the controller and configured to change one of a pressure and a flow rate of the drive gas to a first value if the first control signal is received from the controller and change the one of the pressure and the flow rate of the drive gas to a second value if the second control signal is received from the controller, wherein the second value is greater than the first value. Further, the rotary pump is configured to deliver the drive gas to cause supply of a medical gas during the inspiration time, wherein the medical gas is supplied based on the one of the pressure and the flow rate of the drive gas delivered from the rotary pump.

In accordance with another embodiment useful for understanding the invention, a method for providing respiratory support to a patient is presented. The method includes generating, by a controller, a first control signal for a first time-period and a second control signal for a second time-period during an inspiration time of a ventilation cycle. Also, the method includes delivering, by a rotary pump, a drive gas to a bellow assembly. Further, the method includes changing, by the rotary pump, one of a pressure and a flow rate of the drive gas to a first value if the first control signal is received from the controller. In addition, the method includes changing, by the rotary pump, the one of the pressure and the flow rate of the drive gas to a second value if the second control signal is received from the controller, wherein the second value is greater than the first value. Furthermore, the method includes supplying, by the bellow assembly, a medical gas to the patient during the inspiration time based on the one of the pressure and the flow rate of the drive gas received from the rotary pump.

Further disclosed is a ventilator system for providing respiratory support The ventilator system includes a controller configured to generate a first control signal for a first time-period during an inspiration time of a ventilation cycle. Also, the ventilator system includes a rotary pump electrically coupled to the controller and configured to change one of a pressure and a flow rate of the drive gas to a first value based on the first control signal received from the controller. Further, the controller is configured to generate a second control signal for a second time-period during the inspiration time of the ventilation cycle. In addition, the rotary pump is configured to change the one of the pressure and the flow rate of the drive gas to a second value based on the second control signal received from the controller. The rotary pump is further configured to deliver the drive gas to cause supply of a medical gas during the inspiration time, wherein the medical gas is supplied based on the one of the pressure and the flow rate of the drive gas delivered from the rotary pump. Also, the controller is configured to generate a third control signal for a third time-period based on a pressure signal to maintain a pressure of the medical gas to a desired pressure value, wherein the third control signal is generated during an expiration time of the ventilation cycle. Additionally, the rotary pump is configured to change the pressure of the drive gas to a third value based on the third control signal received from the controller. Also, the rotary pump is further configured to deliver the drive gas to maintain the pressure of the medical gas at the desired pressure value during the expiration time of the ventilation cycle.

### DRAWINGS

These and other features, aspects, and advantages of the present disclosure will become better understood when the following detailed description is read regarding the accompanying drawings in which like characters represent like parts throughout the drawings, wherein:
FIG. 1 is a block diagram of a ventilator system for providing respiratory support to a patient, in accordance with aspects of the present specification;
FIG. 2 is a diagrammatical representation of the ventilator system, in accordance with aspects of the present specification;
FIG. 3 is a block diagram of a controller employed in the ventilator system to control one or more parameters of medical gases, in accordance with aspects of the present specification;
FIG. 4 is a time chart of the ventilator system operated in a volume control ventilation (VCV) mode, in accordance with aspects of the present specification;
FIG. 5 is a time chart of the ventilator system operated in a pressure control ventilation (PCV) mode, in accordance with aspects of the present specification; and
FIG. 6 is a flow chart illustrating a method for providing respiratory support to the patient, in accordance with aspects useful for understanding the invention.

### DETAILED DESCRIPTION

As will be described in detail hereinafter, various embodiments of systems and methods for providing respiratory support to a patient are presented. The systems and methods presented herein employ a rotary pump and a controller to control one or more parameters of medical gas supplied to the patient. Moreover, the rotary pump may use ambient air as a drive gas for driving the medical gas to the patient. As the ambient air is used as the drive gas, pressurized supply tanks are used only for supplying the medical gas. This in turn reduces the usage of the pressurized supply tanks and reduces an operating cost of the system.

In the following specification and the claims, the singular forms "a", "an" and "the" include plural referents unless the context clearly dictates otherwise. As used herein, the term "or" is not meant to be exclusive and refers to at least one of the referenced components being present and includes instances in which a combination of the referenced components may be present, unless the context clearly dictates otherwise.

As used herein, the terms "may" and "may be" indicate a possibility of an occurrence within a set of circumstances; a possession of a specified property, characteristic or function; and/or qualify another verb by expressing one or more of an ability, capability, or possibility associated with the qualified verb. Accordingly, usage of "may" and "may be" indicates that a modified term is apparently appropriate, capable, or suitable for an indicated capacity, function, or usage, while considering that in some circumstances, the modified term may sometimes not be appropriate, capable, or suitable.

In some embodiments, a ventilator system for providing respiratory support is presented. The ventilator system includes a controller configured to generate a first control signal for a first time-period and a second control signal for a second time-period during an inspiration time of a ventilation cycle. Also, the ventilator system includes a rotary pump electrically coupled to the controller and configured to change one of a pressure and a flow rate of the drive gas to a first value if the first control signal is received from the controller. Further, the rotary pump is configured to change the one of the pressure and the flow rate of the drive gas to a second value if the second control signal is received from the controller, wherein the second value is greater than the first value. Furthermore, the rotary pump further is configured to deliver the drive gas to cause supply of a medical gas during the inspiration time, wherein the medical gas is supplied based on the one of the pressure and the flow rate of the drive gas delivered from the rotary pump.

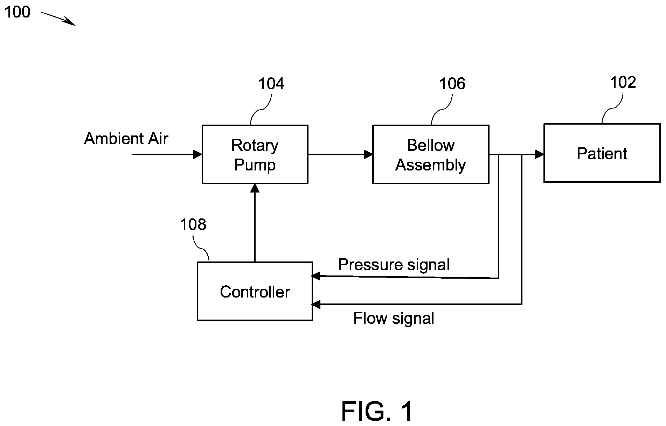

Turning now to the drawings and referring to FIG. 1, a block diagram of a ventilator system 100 for providing respiratory support to a patient 102, in accordance with aspects of the present specification, is depicted. The ventilator system 100 is used for mechanical delivery of a medical gas to the patient 102. In one example, the medical gas includes air, oxygen, nitrogen, helium, nitrous oxide, anesthetic agent, drug aerosol, and/or any other gas breathed by the patient.

In general, the ventilator system 100 is operated in a cycle known as a respiratory cycle or a ventilation cycle that includes an inhalation phase and an exhalation phase. In the inhalation phase, the ventilator system 100 is operated to deliver medical gas under a pressure that is sufficient to overcome patient's airway resistance to fill lungs of the patient 102. It may be noted that a time duration of the inhalation phase is referred to as an "inspiration time" of the ventilation cycle. Further, in the exhalation phase, the ventilator system 100 is operated to reduce the pressure of the medical gas, which in turn causes a pressure difference between the delivered medical gas in the lungs of the patient 102 and the medical gas in the ventilator system 100. As a result, the delivered medical gas in the patient 102 flows out of the patient 102. It may be noted that a time duration of the exhalation phase is referred to as an "expiration time" of the ventilation cycle. Also, the ventilation cycle may be repeated to continuously deliver breath to the patient 102.

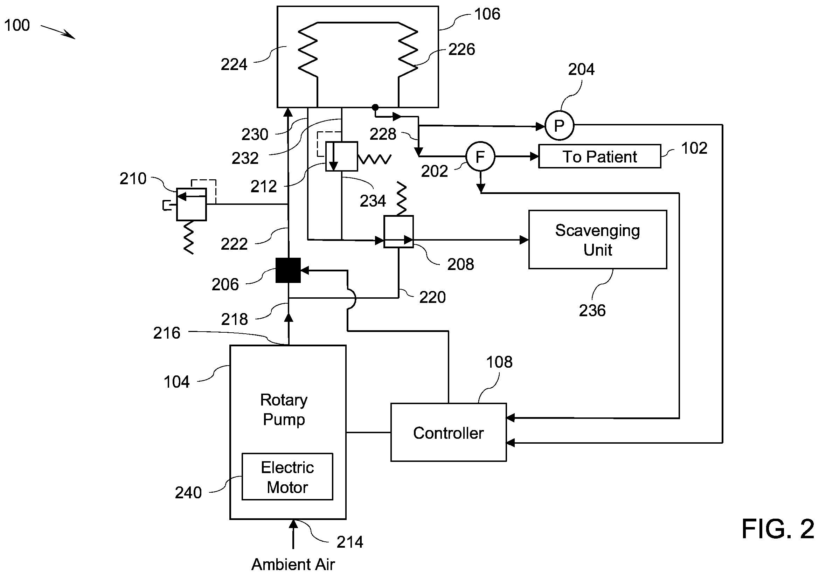

In the presently contemplated configuration, the ventilator system 100 may be operated in a volume control ventilation (VCV) mode or a pressure control ventilation (PCV) mode based on the requirement or condition of the patient 102. The VCV mode is referred to as a mode of operating the ventilator system 100 to control a flow rate of the medical gas supplied to the patient 102. Similarly, the PCV mode is referred to as a mode of operating the ventilator system 100 to control a pressure of the medical gas supplied to the patient 102. The aspect of operating the ventilator system 100 in the VCV mode or the PCV mode is explained in greater detail with reference to FIG. 2. It may be noted that the ventilator system 100 may be operated in other modes, and is not limited to the VCV and PCV modes. Also, the method for providing the respiratory support using the ventilator system 100 may be performed in other modes, and is not limited to the VCV and PCV modes.

As depicted in FIG. 1, the ventilator system 100 includes a rotary pump 104, a bellow assembly 106, and a controller 108. It may be noted that the ventilator system 100 may include other components and is not limited to the components shown in FIG. 1. The rotary pump 104 is operatively coupled to the bellow assembly 106 and the controller 108. In one example, the rotary pump 104 may be a centrifugal pump, an axial pump, a positive displacement pump, or combinations thereof. The rotary pump 104 is configured to pressurize ambient air and supply the pressurized ambient air as drive gas to the bellow assembly 106. It may be noted that the drive gas may be referred to as gas that is used for forcing or pushing the medical gas from the bellow assembly 106 to the patient 102. Also, the drive gas may be used for actuating an exhaust valve (shown in FIG. 2). In one embodiment, the drive gas may include other gases and is not limited to the ambient air as shown in FIG. 1. In one example, the rotary pump 104 includes a centrifugal blower that is operated at a desired rotary speed to supply a desired volumetric flow rate of the drive gas at a desired pressure to the bellow assembly during the inhalation phase. Also, the rotary speed of the blower may be varied to change the flow rate and/or pressure of the drive gas over the course of the inhalation phase. It may be noted that the terms "rotary speed" and "speed" may be used interchangeably in the below description.

In a presently contemplated configuration, the bellow assembly 106 is operatively coupled to the rotary pump 104 and the patient 102. Also, the bellow assembly 106 is configured to receive a flow of the drive gas from the rotary pump 104. Further, this flow of drive gas actuates or compresses bellows (shown in FIG. 2) in the bellow assembly 106 so that medical gas within the bellows is supplied to the patient 102.

Further, the controller 108 is configured to generate one or more control signals to control one or more parameters of the drive gas supplied to the bellow assembly 106. In general, the parameters of the drive gas are controlled to control the flow of medical gas supplied to the patient 102. The parameters of the drive gas may include a pressure of the drive gas and a flow rate of the drive gas. In one example, the controller 108 may include one or more microprocessors or microcontrollers capable of performing parallel processing. Further, as depicted in FIG. 1, the controller 108 may receive a pressure signal and a flow signal from sensors positioned at suitable locations between the bellow assembly 106 and the patient 102. The pressure signal may indicate a pressure of the medical gas supplied from the bellow assembly 106 to the patient 102. Similarly, the flow signal may indicate a flow rate of the medical gas supplied from the bellow assembly 106 to the patient 102. The controller 108 uses the pressure signal and/or the flow signal along with a pre-stored data to control the parameters of the drive gas. In one example, the pre-stored data may include clinician input data, such as respiration rate, a ratio of the inspiration time to the expiration time (I:E ratio), a Positive End Expiratory Pressure (PEEP) value, a tidal volume etc. In one embodiment, the pre-stored data may include other ventilator inputs such as previously provided inputs by the clinician or user to adjust one or more controls of the ventilator system 100.

During the ventilation cycle of the ventilator system 100, the controller 108 generates first and second control signals to control the pressure and/or flow rate of the drive gas produced by the rotary pump 104. In particular, the controller 108 generates a first control signal for a first time-period at the beginning of the inspiration time of the ventilation cycle. Further, the controller 108 generates a second control signal for a second time-period that is after the first time-period during the inspiration time of the ventilation cycle. In one example, the first time-period may be in a range from about 5 ms to about 200 ms. Similarly, the second time-period may be in a range from about 0.25 s to about 5 s.

Further, the controller 108 transmits the first control signal to the rotary pump 104 for the first time-period. The first control signal is a pulse width modulation (PWM) signal having a constant pulse width that is used to run the rotary pump 104 at an intermediate speed for the first time-period. In one embodiment, the rotary pump 104 may include a brushless direct current (BLDC) motor. The controller 108 may have poor performance to directly ramp up the rotary pump 104 to the required speed to supply the drive gas at a desired pressure value or a desired flow rate value to the bellow assembly 106. To overcome this problem, the PWM signal having the constant pulse width is provided to the BLDC motor to run the BLDC motor at the intermediate speed for the first time-period. By running the BLDC motor at the intermediate speed, the controller 108 may have better performance to ramp up the BLDC motor from the intermediate speed to the required speed instantaneously for delivering the drive gas at the desired pressure value or the desired flow rate value to the bellow assembly 106. Also, by operating the BLDC motor at the constant speed, the rotary pump 104 may change the pressure and/or the flow rate of the drive gas to a first value. In one example, the rotary pump 104 includes the centrifugal blower that is operated by the BLDC motor at the constant speed so that the pressure and/or the flow rate of the drive gas is increased to the first value. For example, the pressure of the drive gas is increased from 0 to 30 cm H₂0. Similarly, the flow rate of the drive gas is increased from 0 to 50 lpm.

After the first time-period of the inspiration time, the controller 108 transmits the second control signal to the rotary pump 104. The second control signal is a pulse width modulation (PWM) signal having a varying pulse width that is used to vary the speed of the rotary pump 104. The controller 108 uses the pressure signal and/or the flow signal along with the pre-stored data to generate the second control signal.

Upon receiving the second control signal, the rotary pump 104 is operated to change the pressure or the flow rate of the drive gas to a second value within a shorter time-period. In one example, the shorter time-period is about 50 ms. It may be noted that the second value of the drive gas may correspond to a predetermined value of the medical gas provided by a clinician or an operator to the ventilator system 100. Also, the second value of the drive gas is modified to maintain the predetermined value of the medical gas. In one example, the pulse width of the PWM signal is varied to modify the second value of the drive gas and maintain the predetermined value of the medical gas till the end of the second time-period. Moreover, the second value is greater than the first value. For example, in the PCV mode, the pressure of the drive gas is increased to a value that is in a range from about 10 cm H₂0 to about 80 cm H₂0. Similarly, in the VCV mode, the flow rate of the drive gas is increased to a value that is in a range from about 10 lpm to about 100 lpm. Further, the rotary pump 104 may supply this drive gas to the bellow assembly 106. Based on the change in the pressure or the flow rate of the drive gas, the bellow assembly 106 is configured to supply the medical gas to the patient 102 during the inspiration time of the ventilation cycle. More specifically, with the increase in the pressure and/or the flow rate of the drive gas, the bellows in the bellow assembly 106 is compressed so that the medical gas within the bellows is delivered to the patient 102. Also, the pressure of this medical gas may be sufficient to overcome patient's airway resistance to fill lungs of the patient 102.

During the expiration time of the ventilation cycle, the controller 108 may generate and transmit a third control signal to the rotary pump 104 to reduce the pressure of the drive gas to a third value. The controller 108 may use the pressure signal at the end of the inspiration time to generate the third control signal. In one example, the speed of the rotary pump 104 is decreased to reduce the pressure and/or the flow rate of the drive gas. Also, the drive gas is stopped from flowing to the bellow assembly 106. As a result, the pressure of the medical gas in the bellows is reduced, which in turn causes a pressure difference between the delivered medical gas in the lungs of the patient 102 and the medical gas in the ventilator system 100. As a result, the delivered medical gas in the patient 102 flows out of the patient 102 and may flow back to the bellows. In one embodiment, the drive gas having the pressure of the third value is supplied to an exhaust valve (shown in FIG. 2) to reduce the pressure of the medical gas in the lungs to a desired pressure value or the PEEP value. This pressure of the medical gas in the lungs serves to keep the patient's lungs partially inflated and open. Furthermore, after the expiration time, the ventilator system 100 moves to a next ventilation cycle to repeat inhalation and exhalation of the patient. Also, the ventilation cycle may be repeated to continuously deliver breath to the patient.

Referring to FIG. 2, a diagrammatical representation of the ventilator system 100, in accordance with some aspects of the present specification, is depicted. The ventilator system 100 includes a rotary pump 104, a controller 108, a bellow assembly 106, a flow sensor 202, a pressure sensor 204, an electrical valve 206, an exhaust valve 208, an overpressure valve 210, and a pop-off valve 212.

The rotary pump 104 is configured to receive ambient air via an inlet 214 and convey the pressurized ambient air as a drive gas via an outlet 216 of the rotary pump 104. In one example, an intake filter may be placed at the inlet 214 of the rotary pump 104 to filter the ambient air. As depicted in FIG. 2, the rotary pump 104 is operatively coupled to the electrical valve 206 and the exhaust valve 208. In one example, the rotary pump is coupled to the electrical valve 206 via a first conduit 218 and to the exhaust valve 208 via a portion of the first conduit 218 and a second conduit 220. Also, the rotary pump 104 is electrically coupled to the controller 108. In one embodiment, the rotary pump 104 includes an electric motor 240 that is configured to receive one or more control signals from the controller 108. Further, based on the received control signals, the electric motor 240 may vary the speed of the rotary pump 104 to control one or more parameters of the drive gas. The parameters may include a pressure of the drive gas and a flow rate of the drive gas. In one example, the electric motor 240 may be a BLDC motor.

Further, the electrical valve 206 is configured to convey the drive gas to the bellow assembly 106. As depicted in FIG. 2, the electrical valve 206 is coupled to the bellow assembly 106 via a third conduit 222. Also, the electrical valve 206 is electrically coupled to the controller 108 to receive an activation signal or a deactivation signal. In particular, the electrical valve 206 is activated or opened if the activation signal is received from the controller 108. Upon activating or opening the electrical valve 206, the drive gas that is supplied from the rotary pump 104 is conveyed to the bellow assembly 106. Similarly, the electrical valve 206 is deactivated or closed if the deactivation signal is received from the controller 108. Upon deactivating or closing the electrical valve 206, the supply of drive gas to the bellow assembly 106 is ceased or stopped. In one example, the electrical valve 206 may include a solenoid valve. It may be noted that activation and deactivation of the electrical valve 206 is controlled by the controller 108 based on the ventilation cycle of the ventilator system 100.

Furthermore, the overpressure valve 210 is operatively coupled to the third conduit 222 to maintain the pressure of the drive gas within a threshold pressure value. In particular, the overpressure valve 210 is used to vent any excess pressure of the drive gas to the ambient air so that the safety of the patient 102 is maintained during ventilation. It may be noted that the excess pressure may be any pressure that is above the threshold pressure value. In one example, the threshold pressure value may be in a range from about 75 cmH₂O to 100 cmH₂O.

In the illustrated embodiment, the bellow assembly 106 includes a bellows chamber 224 and a bellows 226. As depicted in FIG. 2, the bellows chamber 224 is operatively coupled to the third conduit 222 to receive the drive gas. Also, the bellows 226 is used to store the medical gas that needs to be delivered to the patient 102. In one embodiment, the bellows 226 may receive the medical gas from an external gas storage unit (not shown) via an anesthetic and humidifier unit (not shown). In one example, one or more flow control valves may be used between the external gas storage unit (not shown) and the anesthetic and humidifier unit (not shown) to control a flow of medical gas to the bellows 226. Further, the bellows 226 may compress or expand based on pressure of the drive gas in the bellows chamber 224. In one example, the external gas storage unit may include one or more pressurized supply tanks. Since the ambient air is used as the drive gas and the pressurized supply tanks are used for supplying only the medical gas, the pressurized supply tanks may be used for a longer period. As a result, operating cost of the ventilator system 100 is substantially reduced.

Further, in the inspiration time of the ventilation cycle, the drive gas that is received from the third conduit 222 may pressurize the bellows chamber 224 and compresses the bellows 226 to direct the medical gas within the bellows 226 to the patient 102 via a fourth conduit 228. In one embodiment, the fourth conduit 228 may be coupled to the patient 102 through a patient interface unit (not shown) to deliver the medical gas from the bellows 226 to the patient 102. In a similar manner, in the expiration time of the ventilation cycle, the pressure of the medical gas in bellows 226 is reduced, which in turn causes a pressure difference between the delivered medical gas in the lungs of the patient 102 and the medical gas in the ventilator system 100. As a result, the delivered medical gas in the patient 102 flows out of the patient 102 and may flow back to the bellows 226 via the fourth conduit 228. As a result, the bellows 226 may expand within the bellow assembly 106 to displace or release the drive gas from the bellows chamber 224 to the exhaust valve 208 via a fifth conduit 230 that is coupled between the bellow assembly and the exhaust valve 208. Also, during the expiration time, a portion of the medical gas in the bellows 226 may be released to the pop-off valve 212 via a sixth conduit 232 that is coupled between the bellows 226 and the pop-off valve 212. Moreover, if the patient lung pressure is above the set PEEP and the bellows 226 is at its topmost position and unable to move further in the bellow assembly 106, the pop-off valve 212 opens to convey this portion of the medical gas to the exhaust valve 208 via a seventh conduit 234 that is coupled between the pop-off valve 212 and the exhaust valve 208.

In a presently contemplated configuration, the exhaust valve 208 is used to direct any medical gas from the pop-off valve 212 and the drive gas from the bellows chamber 224 to a scavenging unit 236. Further, the scavenging unit 236 is configured to remove any medical gases that may be harmful to clinicians or others in the room with the patient 102 if these medical gases were allowed to exhaust into the room. In one embodiment, the scavenging unit 236 vents the medical gases out of the hospital or care facility where these medical gases are diluted to non-harmful concentrations in the environment.

In addition, the exhaust valve 208 may be configured to control the pressure of the gas in the bellows 226 and correspondingly the pressure of the medical gas in the patient's lungs. It may be noted that the pressure of the medical gas in the patient's lungs at the end of the expiration time is known as a positive end expiratory pressure (PEEP). In particular, the pressure of the drive gas supplied to the exhaust valve 208 is maintained at the PEEP value so that the patient's exhalation reaches a pressure equilibrium with the medical gas within the bellows 226 at the PEEP, thus maintaining the pressure within the patient's lungs. In one embodiment, by controlling the PEEP during patient exhalation, the patient's lungs are not returned to the ambient pressure, but instead are held at a pressure above the ambient pressure that is determined by the clinician. The PEEP serves to keep the patient's lungs partially inflated and open.

Furthermore, the pressure sensor 204 may be coupled to the bellow assembly 106 to determine the pressure of the medical gas supplied from the bellows 226 to the patient 102. In the embodiment of FIG. 2, the pressure sensor 204 may be coupled to the fourth conduit 228. Further, the pressure sensor 204 may transmit a pressure signal to the controller 108. The pressure signal indicates or represents the pressure of the medical gas supplied from the bellows 226 to the patient 102. In a similar manner, the flow sensor 202 may be coupled to the bellow assembly 106 to determine the volume of the medical gas supplied from the bellows 226 to the patient 102. It may be noted that the volume of the medical gas per unit time supplied from the bellows is referred to as a flow rate of the medical gas. In the embodiment of FIG. 2, the flow sensor 202 may be coupled to the fourth conduit 228. Further, the flow sensor 202 may transmit a flow signal to the controller 108. The flow signal indicates or represents the flow rate of the medical gas supplied from the bellows 226 to the patient 102.

During the operation of the ventilator system 100, the controller 108 may begin the ventilation cycle by generating the first control signal for the first time-period of the inspiration time. The controller 108 generates the first control signal based on the pre-stored data. In one example, the pre-stored data may include information that is associated with the patient 102. Some of the information includes respiration rate, a ratio of the inspiration time to the expiration time (I:E ratio), a Positive End Expiratory Pressure (PEEP) value, and a tidal volume. In one embodiment, the clinician may provide this information as per the requirement of the patient 102.

Further, the controller 108 transmits the first control signal to the rotary pump 104 to operate the rotary pump 104 at an intermediate speed for the first time-period. The first control signal is a pulse width modulation (PWM) signal having a constant pulse width. In one embodiment, the controller 108 may have poor performance to directly ramp up the rotary pump 104 to the required speed to supply the drive gas at a desired pressure value or a desired flow rate value to the bellow assembly 106. To overcome this problem, the PWM signal having the constant pulse width is provided to the electric motor to run the electric motor at the intermediate speed for the first time-period. By running the electric motor at the intermediate speed, the controller 108 may have better performance to ramp-up the electric motor from the intermediate speed to the required speed for delivering the drive gas at the desired pressure value or the desired flow rate value to the bellow assembly 106.

Also, by operating the rotary pump 104 at the constant speed, the pressure and/or flow rate of the drive gas is changed to the first value. Further, the controller 108 may concurrently or alternately transmit the activation signal to the electrical valve 206 to activate or open the electrical valve 206 so that the drive gas is conveyed from the rotary pump 104 to the bellow assembly 106.

After operating the rotary pump 104 at the constant speed for the first time-period, the controller 108 generates the second control signal for the second time-period. The second time-period is after the first time-period of the inspiration time. In particular, the controller 108 may receive the pressure signal indicating the pressure of the medical gas from the pressure sensor 204. Also, the controller 108 may receive the flow signal indicating the flow rate of the medical gas from the flow sensor 202. Further, the controller 108 generates the second control signal based on the pressure and/or the flow rate of the medical gas supplied to the patient 102. More specifically, if the ventilator system 100 is operated in the VCV mode, the controller 108 generates the second control signal based on the flow rate of the medical gas supplied to the patient 102. Similarly, if the ventilator system 100 is operated in the PCV mode, the controller 108 generates the second control signal based on the pressure of the medical gas supplied to the patient 102.

Further, the controller 108 transmits the second control signal to the rotary pump 104 to change the pressure and/or flow rate of the drive gas to the second value. It may be noted that the second value of the drive gas may correspond to a predetermined value of the medical gas provided by a clinician or an operator to the ventilator system 100. Also, the second value of the drive gas is modified to maintain the predetermined value of the medical gas. More specifically, if the ventilator system 100 is operated in the VCV mode, the rotary pump 104 is operated to change the flow rate of the drive gas from the first value to the second value. In a similar manner, if the ventilator system 100 is operated in the PCV mode, the rotary pump 104 is operated to the change the pressure of the drive gas from the first value to the second value. The second control signal is a PWM signal that is transmitted to the electric motor 240 in the rotary pump 104. The PWM signal is used to vary the rotary speed of the rotary pump 104 so as to rapidly increase the pressure and/or flow rate of the drive gas to the second value. Also, the PWM signal is varied to modify the second value of the drive gas and maintain the predetermined value of the medical gas till the end of the second time-period.

As the pressure and/or flow rate of the drive gas is increased to the second value, the drive gas supplied to the bellow assembly 106 is pressurized in the bellows chamber 224. As a result, the bellows 226 are compressed to direct the medical gas within the bellows 226 to the patient 102 via the fourth conduit 228. The sum of the first and second time-periods may correspond to total inhalation time set by the clinician. It may be noted that any excess pressure of the drive gas is released to the ambient air via the overpressure valve 210 to maintain the safety of the patient 102 during the inspiration time of the ventilation cycle.

At the end of the inspiration time, the controller 108 may receive the pressure signal from the pressure sensor 204. Further, at the beginning of the expiration time of the ventilation cycle, the controller 108 may generate the third control signal based on the pressure of the medical gas supplied to the patient 102. Also, the controller 108 transmits the deactivation signal to the electrical valve 206 to deactivate or close the electrical valve 206 so as to cease the flow of drive gas to the bellow assembly 106. Additionally, upon closing the electrical valve 206, the drive gas is supplied from the rotary pump 104 to the exhaust valve 208 via the portion of the first conduit 218 and the second conduit 220, as depicted in FIG. 2.

Furthermore, the controller 108 transmits the third control signal to the rotary pump 104 to change the pressure of the drive gas to the third value. It may be noted that the third value of the drive gas is same as the PEEP value or a desired pressure value of the medical gas supplied to the patient 102. More specifically, the controller 108 transmits the third control signal to the rotary pump 104 to reduce the pressure of the medical gas in the patient 102 to the PEEP value or the desired value. Also, the exhaust valve 208 receives the drive gas having the pressure of the third value from the rotary pump 104. Further, the exhaust valve 208 maintains the pressure of the medical gas at the desired pressure value as a result of the drive gas having the pressure of the third value received from the rotary pump 104. Therefore, the pressure of the medical gas is maintained at the desired pressure value during the expiration time of the ventilation cycle. In one embodiment, the third control signal may be a PWM signal that is used to vary the speed of the rotary pump 104 so that the pressure of the drive gas is reduced from the second value to the third value.

At the end of the expiration time of the ventilation cycle, the controller 108 along with the exhaust valve 208 reduces the pressure of the medical gas to the PEEP value or the desired value. In one embodiment, if the ventilator system 100 is operated in the VCV mode, the controller 108 continues to generate the third control signal till the end of the expiration time to maintain the pressure of the medical gas to the PEEP value or the desired value. However, if the ventilator system 100 is operated in the PCV mode, the pressure of the medical gas is reduced to the PEEP value or the desired value at least before a fourth time-period at an end of the expiration time of the ventilation cycle. In one example, the fourth time-period is in a range from about 0 to about 50 ms. In one embodiment, the electric motor 240 in the rotary pump 104 may be unable to ramp up to a predefined speed instantaneously. As a consequence, the rotary pump 104 may have a slow response time to supply the drive gas at a desired pressure value or a desired flow rate value to the bellow assembly 106 during the inspiration time. To improve a response time of the rotary pump 104, the controller 108 generates a fourth control signal for the fourth time-period at the end of the expiration time. Further, the rotary pump 104 receives the fourth control signal and changes the pressure of the drive gas to a fourth value. The fourth value is greater than the third value and less than the first value. Also, when the ventilator system 100 starts the next ventilation cycle, the controller 108 generates the first control signal to increase the pressure of the drive gas from the fourth value to the first value. As a result, the electric motor 240 in the rotary pump 104 is already operating at an intermediate speed corresponding to the fourth value and therefore electric motor 240 takes shorter time-period to go to the speed required to increase the pressure of the drive gas to the second value. In one example, the shorter time-period may be about 50 ms.

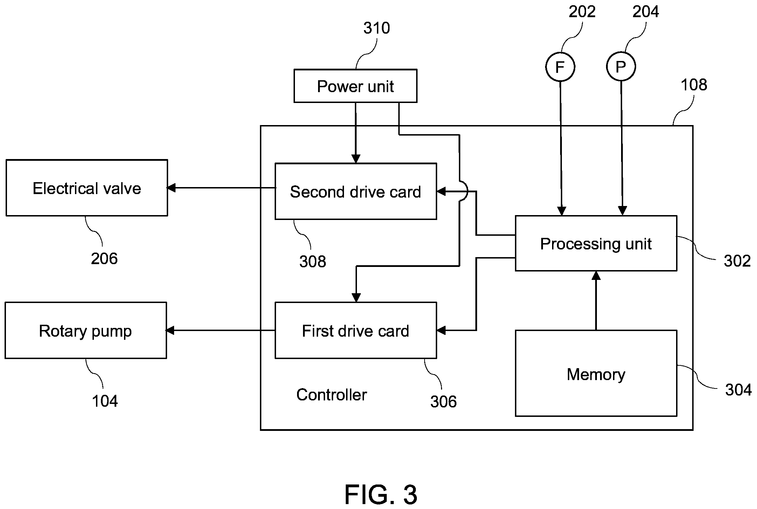

Turing now to FIG. 3, a block diagram of a controller 108 employed in the ventilator system 100 to control one or more parameters of medical gases, in accordance with aspects of the present specification, is depicted. The controller 108 includes a processing unit 302, a memory 304, a first drive card 306, and a second drive card 308. The memory 304 includes pre-stored data associated with the patient 102. Also, the memory 304 may be used to store other data provided by the clinician or the user via an interface unit (not shown). Further, the memory 304 is electrically coupled to the processing unit 302.

As depicted in FIG. 3, the processing unit 302 is electrically coupled to the first drive card 306 and the second drive card 308. Also, the processing unit 302 is electrically coupled to the flow sensor 202 and the pressure sensor 204. It may be noted that the processing unit 302 and/or the first drive card 306 may include a proportional-integral-derivative (PID) controller that is used to generate PWM signals. In one embodiment, the processing unit 302 may receive power supply from a power unit 310 that is positioned external to the controller 108. It may be noted that the power unit 310 may be positioned within the controller 108 or outside the controller 108. In one example, the processing unit 302 may receive a voltage that is in a range from about 1 V to about 10 V from the power unit 310.

In a similar manner, the first drive card 306 and the second drive card 308 may receive the power supply from the power unit 310. In one example, the first and second drive cards 306, 308 may receive a voltage that is in a range from about 10 V to about 24 V from the power unit 310. Also, the first drive card 306 is electrically coupled to the rotary pump 104 and configured to drive or control the speed of the rotary pump 104. Similarly, the second drive card 308 is electrically coupled to the electrical valve 206 and configured to activate and deactivate the electrical valve 206.

In one embodiment, the processing unit 302 generates a first low voltage signal. In one example, the magnitude of the first low voltage signal is in a range from about 1 V to about 8V. Further, the processing unit 302 transmits the first low voltage signal to the first drive card 306. Also, the first drive card 306 receives the voltage from the power unit 310. In one example, the magnitude of the voltage received from the power unit 310 is about 24 V. Thereafter, the first drive card 306 generates a PWM signal having a varying pulse width based on the first low voltage signal and the voltage received from the power unit 310. More specifically, the pulse width of the PWM signal is varied based on the magnitude of the first low voltage signal received from the processing unit 310. Also, the magnitude of the PWM signal is selected corresponding to the magnitude of the voltage received from the power unit 310. Furthermore, the first drive card 306 transmits the generated PWM signal having the varying pulse width as a control signal to the rotary pump 104 to control the speed of the rotary pump 104. The control signal may be one of the first control signal, the second control signal, the third control signal, and the fourth control signal as referred to in FIG. 1.

In another embodiment, the processing unit 302 generates a PWM signal having a varying pulse width. In one example, the magnitude of the PWM signal is about 5 V. Further, the PWM signal having the varying pulse width is transmitted to the first drive card 306. Also, the first drive card 306 receives the voltage from the power unit 310. In one example, the magnitude of the voltage received from the power unit 310 is about 24 V. Thereafter, the first drive card 306 amplifies or increases the magnitude of the PWM signal that is corresponding to the magnitude of the voltage received from the power unit 310. In one example, the magnitude of the PWM signal varies from 5 V to 24 V. Furthermore, the first drive card 306 transmits the amplified PWM signal having the varying pulse width and the varying magnitude as a control signal to the rotary pump 104 to control the speed of the rotary pump 104.

In yet another embodiment, the processing unit 302 generates and transmits a PWM signal having a predefined or constant pulse width to the first drive card 306. Further, the first drive card 306 varies the magnitude of the PWM signal based on the magnitude of the voltage received from the power unit. 310. Furthermore, the first drive card 306 transmits the PWM signal having a varying magnitude and constant pulse width as a control signal to the rotary pump 104 to control the speed of the rotary pump 104.

In a similar manner, the processing unit 302 generates a second low voltage signal. In one example, the magnitude of the second low voltage signal is in a range from about 0 V to about 5V. Also, the processing unit 302 transmits the second low voltage signal to the second drive card 308. Further, the second drive card 308 uses the voltage received from the power unit 310 to amplify the second low voltage signal and transmits the amplified second low voltage signal as an activation signal or a deactivation signal to the electrical valve 206 to open or close the electrical valve 206. In one example, the magnitude of the activation signal or the deactivation signal is in a range from about 0 V to about 24 V.

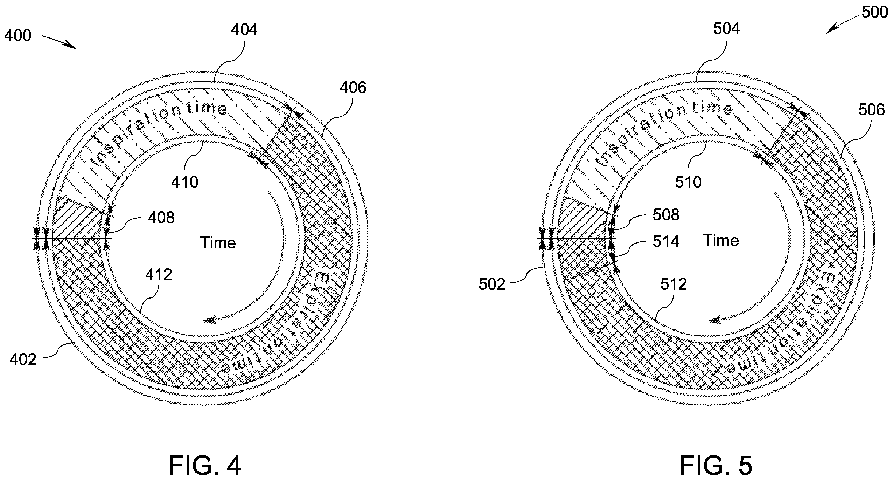

Referring to FIG. 4, a time chart 400 of a ventilator system 100 operated in a volume control ventilation (VCV) mode, in accordance with aspects of the present specification, is depicted. Reference numeral 402 represents the ventilation cycle of the ventilator system 100. Further, reference numeral 404 represents an inspiration time of the ventilation cycle 402. Also, reference numeral 406 represents an expiration time of the ventilation cycle 402.

Further, reference numeral 408 represents the first time-period of the inspiration time 404. In the first time-period 408, the controller 108 generates a first control signal to operate the rotary pump 104 at a constant speed. Also, the flow rate of the drive gas is increased to a first value. Similarly, reference numeral 410 represents the second time-period of the inspiration time 404. In the second time-period 410, the controller 108 generates a second control signal to increase and maintain the flow rate of the drive gas to a second value.

In the expiration time 406 of the ventilation cycle 402, reference numeral 412 represents a third time-period where the controller 108 generates a third control signal to reduce the pressure of the drive gas to a third value. Also, in the third time-period 412, the pressure of the medical gas supplied to the patient 102 is reduced or maintained at a desired pressure value or a PEEP value.

Referring to FIG. 5, a time chart 500 of a ventilator system 100 operated in a pressure control ventilation (PCV) mode, in accordance with aspects of the present specification, is depicted. Reference numeral 502 represents the ventilation cycle of the ventilator system 100. Further, reference numeral 504 represents an inspiration time of the ventilation cycle 502. Also, reference numeral 506 represents an expiration time of the ventilation cycle 502.

Further, reference numeral 508 represents the first time-period of the inspiration time 504. In the first time-period 508, the controller 108 generates a first control signal to operate the rotary pump 104 at a constant speed. Also, the pressure of the drive gas is increased to a first value. Similarly, reference numeral 510 represents the second time-period of the inspiration time 504. In the second time-period 510, the controller 108 generates a second control signal to increase and maintain the pressure of the drive gas to a second value.

In the expiration time 506 of the ventilation cycle 502, reference numeral 512 represents a third time-period where the controller 108 generates a third control signal to reduce the pressure of the drive gas to a third value. Also, in the third time-period 512, the pressure of the medical gas supplied to the patient 102 is reduced or maintained at a desired pressure value or PEEP value. Further, reference numeral 514 represents a fourth time-period at the end of the expiration time 506. In the fourth time-period 514, the controller 108 generates a fourth control signal to change the pressure of the drive gas from the third value to a fourth value. In one example, the fourth value is greater than the third value and less than the first value.

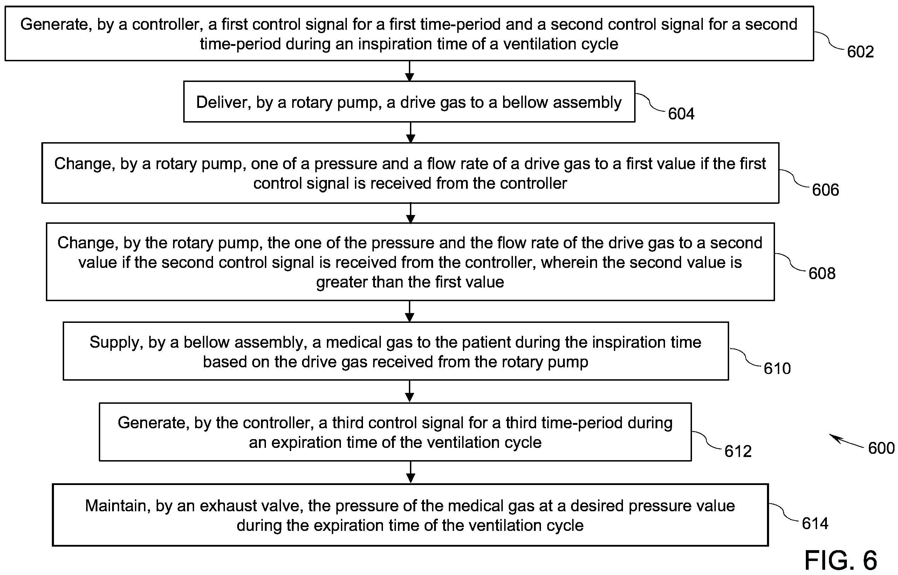

Referring to FIG. 6, a flow chart illustrating a method 600 for providing respiratory support to a patient, in accordance with aspects useful for understanding the invention. For ease of understanding, the method 600 is described with reference to the components of FIGs. 1-3. The method 600 begins with generating, by a controller, a first control signal for a first time-period and a second control signal for a second time-period during an inspiration time of a ventilation cycle, as shown in step 602. The controller may generate the first control signal at the beginning of the inspiration time. In one embodiment, the first control signal is generated based on the pre-stored data associated with the patient. Similarly, the controller may generate the second control signal after the first time-period of the inspiration time. In one embodiment, the second control signal is generated based on the pressure signal and/or the flow signal received by the controller.

Subsequently, at step 604, the method includes delivering, by a rotary pump, a drive gas to a bellow assembly. More specifically, the rotary pump is operatively coupled to the bellow assembly and the controller. Further, the rotary pump is configured to pressurize ambient air and supply the pressurized ambient air as drive gas to the bellow assembly.

In addition, at step 606, the method includes changing, by the rotary pump, one of a pressure and a flow rate of the drive gas to a first value if the first control signal is received from the controller. The rotary pump receives the first control signal for the first time-period of the inspiration time. Further, based on the first control signal, the rotary pump is operated at an intermediate speed to improve the controller performance for delivering a proper supply of the drive gas to the bellow assembly. Also, by operating the rotary pump at the intermediate speed, the rotary pump may change the pressure and/or the flow rate of the drive gas to a first value.

Furthermore, at step 608, the method includes changing, by the rotary pump, the one of the pressure and the flow rate of the drive gas to a second value if the second control signal is received from the controller, wherein the second value is greater than the first value. The rotary pump receives the second control signal for the second time-period of the inspiration time. Further, the rotary pump is operated to change and maintain the pressure and/or the flow rate of the drive gas to a second value. The second value is greater than the first value. Further, the rotary pump may supply this drive gas to the bellow assembly.

In addition, at step 610, the method includes supplying, by the bellow assembly, a medical gas to the patient during the inspiration time based on the one of the pressure and the flow rate of the drive gas received from the rotary pump. More specifically, with the increase in the pressure and/or the flow rate of the drive gas, the bellows in the bellow assembly is compressed so that the medical gas within the bellows is delivered to the patient. Also, the pressure of this medical gas is sufficient to overcome patient's airway resistance to fill the lungs of the patient.

Moreover, at step 612, the method includes generating, by the controller, a third control signal for a third time-period during an expiration time of the ventilation cycle. The controller receives a pressure signal associated with the pressure of the medical gas from the pressure sensor at an end of the inspiration time of the ventilation cycle. Further, the controller generates the third control signal for a third time-period based on the pressure signal to reduce and maintain the pressure of the medical gas to a desired pressure value.

Subsequently, at step 614, the method includes maintaining, by an exhaust valve 208, the pressure of the medical gas at a desired pressure value during the expiration time of the ventilation cycle. More specifically, the rotary pump changes the pressure of the drive gas to a third value based on the third control signal received from the controller. Further, the exhaust valve receives the drive gas having the pressure of the third value from the rotary pump. Thereafter, the exhaust valve maintains or reduces the pressure of the medical gas at the desired pressure value based on the drive gas having the pressure of the third value received from the rotary pump.

The various embodiments of the exemplary systems and methods presented hereinabove aid in providing respiratory support to a patient. Also, the exemplary systems and methods presented hereinabove uses the ambient air as a drive gas for supplying the medical gas to the patient. As the ambient air is used as the drive gas, pressurized supply tanks are used only for supplying the medical gas. This in turn minimizes usage of the pressurized supply tanks and reduces the operating cost of the system.

While only certain features of the present disclosure have been illustrated, and described herein, many modifications and changes will occur to those skilled in the art. The scope of the present invention is limited by the scope of the appended claims.

## Claims

1. A ventilator system for providing respiratory support, the ventilator system comprising:
a controller (108) configured to generate a first control signal for a first time-period based on a pre-stored data, and a second control signal for a second time-period during an inspiration time of a ventilation cycle based on at least one of a pressure and a flow rate of the medical gas supplied from the bellow assembly to the patient during the inspiration time of the ventilation cycle, wherein the second time-period is after the first time-period during the inspiration time and wherein the first control signal is a pulse width modulation signal having a constant pulse width and the second control signal is a pulse width modulation signal having a varying pulse width;
a bellow assembly (106);
a rotary pump (104) electrically coupled to the controller and configured to:
run an intermediate speed to change one of a pressure and a flow rate of a drive gas to a first value if the first control signal is received from the controller; and
vary the speed of the pump to change the one of the pressure and the flow rate of the drive gas to a second value if the second control signal is received from the controller, wherein the second value is greater than the first value; and
the rotary pump further configured to deliver the drive gas to the bellow assembly (106), wherein the bellow assembly is configured to supply a medical gas during the inspiration time based on the one of the pressure and the flow rate of the drive gas delivered from the rotary pump.

2. The ventilator system of claim 1, further comprising:
an electrical valve (206) operatively coupled to the rotary pump and the bellow assembly, and configured to convey the drive gas from the rotary pump to the bellow assembly; and
the controller is configured to activate the electrical valve during the inspiration time of the ventilation cycle to convey the drive gas from the rotary pump to the bellow assembly, and deactivate the electrical valve during an expiration time of the ventilation cycle to cease a flow of the drive gas to the bellow assembly.

3. The ventilator system of claim 1, further comprising:
a pressure sensor (204) coupled to the bellow assembly and configured to determine a pressure of the medical gas supplied from the bellow assembly to a patient; and
a flow sensor (202) coupled to the bellow assembly and configured to determine a flow rate of the medical gas supplied from the bellow assembly to the patient.

4. The ventilator system of claim 3, wherein the controller is further configured to:
receive a pressure signal associated with the pressure of the medical gas from the pressure sensor at an end of the inspiration time of the ventilation cycle;
generate a third control signal for a third time-period based on the pressure signal to maintain the pressure of the medical gas to a desired pressure value, wherein the third control signal is generated during an expiration time of the ventilation cycle.

5. The ventilator system of claim 4, wherein the rotary pump is configured to change the pressure of the drive gas to a third value based on the third control signal received from the controller.

6. The ventilator system of claim 5, wherein the third value of the pressure of the drive gas is less than the second value of the pressure of the drive gas.

7. The ventilator system of claim 6, further comprising an exhaust valve (208) operatively coupled to the rotary pump and the bellow assembly, and configured to:
receive the drive gas having the pressure of the third value from the rotary pump; and
maintain the pressure of the medical gas at the desired pressure value during the expiration time of the ventilation cycle.

8. The ventilator system of claim 7, wherein the controller is configured to generate a fourth control signal for a fourth time-period at an end of the expiration time of the ventilation cycle, wherein the rotary pump is configured to change the pressure of the drive gas to a fourth value based on the fourth control signal received from the controller, and wherein the fourth value is greater than the third value and less than the first value.

## Patentansprüche

1. Beatmungssystem zum Bereitstellen von Beatmungsunterstützung, wobei das Beatmungssystem Folgendes umfasst:
eine Steuerung (108), die ausgelegt ist zum Erzeugen eines ersten Steuersignals für einen ersten Zeitraum basierend auf vorab gespeicherten Daten und eines zweiten Steuersignals für einen zweiten Zeitraum während einer Einatmungszeit eines Beatmungszyklus basierend auf mindestens entweder einem Druck und/oder einer Durchflussrate des medizinischen Gases, das von der Balgenbaugruppe dem Patienten während der Einatmungszeit des Beatmungszyklus zugeführt wird, wobei der zweite Zeitraum nach dem ersten Zeitraum während der Einatmungszeit liegt und wobei das erste Steuersignal ein Pulsbreitenmodulationssignal mit einer konstanten Pulsbreite ist und das zweite Steuersignal ein Pulsbreitenmodulationssignal mit einer variierenden Pulsbreite ist;
eine Balgenbaugruppe (106);
eine Rotationspumpe (104), die elektrisch mit der Steuerung gekoppelt ist und ausgelegt ist zum:
Fahren einer mittleren Geschwindigkeit, um einen Druck und eine Durchflussrate eines Antriebsgases auf einen ersten Wert zu ändern, wenn das erste Steuersignal von der Steuerung empfangen wird; und
Variieren der Pumpendrehzahl, um den Druck und die Durchflussrate des Antriebsgases auf einen zweiten Wert zu ändern, wenn das zweite Steuersignal von der Steuerung empfangen wird, wobei der zweite Wert größer als der erste Wert ist; und
wobei die Rotationspumpe weiterhin ausgelegt ist zum Zuführen des Antriebsgases an die Balgenbaugruppe (106), wobei die Balgenbaugruppe ausgelegt ist zum Zuführen, während der Einatmungszeit, eines medizinischen Gases basierend auf entweder dem Druck oder der Durchflussrate des von der Rotationspumpe zugeführten Antriebsgases.

2. Beatmungssystem nach Anspruch 1, weiterhin Folgendes umfassend:
ein elektrisches Ventil (206), das betriebsfähig mit der Rotationspumpe und der Balgenbaugruppe gekoppelt ist und ausgelegt ist zum Befördern des Antriebsgases von der Rotationspumpe zur Balgenbaugruppe; und
wobei die Steuerung ausgelegt ist zum Aktivieren des elektrischen Ventils während der Einatmungszeit des Beatmungszyklus, um das Antriebsgas von der Rotationspumpe zur Balgenbaugruppe zu befördern, und Deaktivieren des elektrischen Ventils während einer Ausatmungszeit des Beatmungszyklus, um den Fluss des Antriebsgases zur Balgenbaugruppe zu unterbrechen.

3. Beatmungssystem nach Anspruch 1, weiterhin Folgendes umfassend:
einen Drucksensor (204), der mit der Balgenbaugruppe gekoppelt ist und ausgelegt ist zum Bestimmen des Drucks des medizinischen Gases, das von der Balgenbaugruppe einem Patienten zugeführt wird; und
einen Durchflusssensor (202), der mit der Balgenbaugruppe gekoppelt ist und ausgelegt ist zum Bestimmen der Durchflussrate des medizinischen Gases, das von der Balgenbaugruppe dem Patienten zugeführt wird.

4. Beatmungssystem nach Anspruch 3, wobei die Steuerung weiterhin ausgelegt ist zum:
Empfangen eines Drucksignals, das mit dem Druck des medizinischen Gases verknüpft ist, vom Drucksensor am Ende der Einatmungszeit des Beatmungszyklus;
Erzeugen eines dritten Steuersignals für einen dritten Zeitraum basierend auf dem Drucksignal, um den Druck des medizinischen Gases auf einem gewünschten Druckwert zu halten, wobei das dritte Steuersignal während einer Ausatmungszeit des Beatmungszyklus erzeugt wird.

5. Beatmungssystem nach Anspruch 4, wobei die Rotationspumpe ausgelegt ist zum Ändern des Drucks des Antriebsgases auf einen dritten Wert basierend auf dem dritten von der Steuerung empfangenen Steuersignal.

6. Beatmungssystem nach Anspruch 5, wobei der dritte Wert des Drucks des Antriebsgases kleiner ist als der zweite Wert des Drucks des Antriebsgases.

7. Beatmungssystem nach Anspruch 6, ferner umfassend ein Auslassventil (208), das betriebsfähig mit der Rotationspumpe und der Balgenbaugruppe gekoppelt ist und ausgelegt ist zum:
Aufnehmen des Antriebsgases mit dem Druck des dritten Werts von der Rotationspumpe; und
Halten des Drucks des medizinischen Gases während der Ausatmungszeit des Beatmungszyklus auf dem gewünschten Druckwert.

8. Beatmungssystem nach Anspruch 7, wobei die Steuerung ausgelegt ist zum Erzeugen eines vierten Steuersignals für einen vierten Zeitraum am Ende der Ausatmungszeit des Beatmungszyklus, wobei die Rotationspumpe ausgelegt ist zum Ändern des Drucks des Antriebsgases auf einen vierten Wert basierend auf dem von der Steuerung empfangenen vierten Steuersignal, und wobei der vierte Wert größer als der dritte Wert und kleiner als der erste Wert ist.

## Revendications

1. Système de ventilateur pour fournir une assistance respiratoire, le système de ventilateur comprenant :
un dispositif de commande (108) configuré pour générer un premier signal de commande pour une première période de temps basée sur une donnée pré-enregistrée, et un deuxième signal de commande pour une deuxième période de temps pendant un temps d'inspiration d'un cycle de ventilation basé sur au moins l'un d'une pression et d'un débit du gaz médical fourni par l'ensemble de soufflets au patient pendant le temps d'inspiration du cycle de ventilation, la deuxième période étant postérieure à la première période pendant le temps d'inspiration et le premier signal de commande étant un signal de modulation de largeur d'impulsion ayant une largeur d'impulsion constante et le deuxième signal de commande étant un signal de modulation de largeur d'impulsion ayant une largeur d'impulsion variable ;
un ensemble de soufflets (106) ;
une pompe rotative (104) couplée électriquement au dispositif de commande et configurée pour :
faire fonctionner une vitesse intermédiaire pour modifier l'un d'une pression et d'un débit d'un gaz d'entraînement à une première valeur si le premier signal de commande est reçu en provenance du dispositif de commande ; et
faire varier la vitesse de la pompe pour faire modifier la pression ou le débit du gaz d'entraînement à une deuxième valeur si le deuxième signal de commande est reçu en provenance du dispositif de commande, la deuxième valeur étant supérieure à la première valeur ; et
la pompe rotative étant en outre configurée pour fournir le gaz d'entraînement à l'ensemble de soufflets (106), l'ensemble de soufflets étant configuré pour fournir un gaz médical pendant le temps d'inspiration sur la base de la pression et du débit du gaz d'entraînement fourni par la pompe rotative.

2. Système de ventilateur selon la revendication 1, comprenant en outre :
une vanne électrique (206) couplée de manière opérationnelle à la pompe rotative et à l'ensemble de soufflets, et configurée pour acheminer le gaz d'entraînement de la pompe rotative à l'ensemble de soufflets ; et
le dispositif de commande étant configuré pour activer la vanne électrique pendant le temps d'inspiration du cycle de ventilation pour acheminer le gaz d'entraînement de la pompe rotative à l'ensemble de soufflets, et pour désactiver la vanne électrique pendant un temps d'expiration du cycle de ventilation pour arrêter un écoulement du gaz d'entraînement vers l'ensemble de soufflets.

3. Système de ventilateur selon la revendication 1, comprenant en outre :
un capteur de pression (204) couplé à l'ensemble de soufflets et configuré pour déterminer une pression du gaz médical fourni par l'ensemble de soufflets à un patient ; et
un capteur de débit (202) couplé à l'ensemble de soufflets et configuré pour déterminer un débit du gaz médical fourni par l'ensemble de soufflets au patient.

4. Système de ventilateur selon la revendication 3, le dispositif de commande étant en outre configuré pour :
recevoir un signal de pression associé à la pression du gaz médical provenant du capteur de pression à la fin du temps d'inspiration du cycle de ventilation ;
générer un troisième signal de commande pour une troisième période de temps basée sur le signal de pression afin de maintenir la pression du gaz médical à une valeur de pression souhaitée, le troisième signal de commande étant généré pendant un temps d'expiration du cycle de ventilation.

5. Système de ventilateur selon la revendication 4, la pompe rotative étant configurée pour modifier la pression du gaz d'entraînement à une troisième valeur sur la base du troisième signal de commande reçu en provenance du dispositif de commande.

6. Système de ventilateur selon la revendication 5, la troisième valeur de la pression du gaz d'entraînement étant inférieure à la deuxième valeur de la pression du gaz d'entraînement.

7. Système de ventilateur selon la revendication 6, comprenant en outre une soupape d'échappement (208) couplée de manière opérationnelle à la pompe rotative et à l'ensemble de soufflets, et configurée pour :
recevoir le gaz d'entraînement ayant la pression de la troisième valeur à partir de la pompe rotative ; et
maintenir la pression du gaz médical à la valeur de pression souhaitée pendant la durée d'expiration du cycle de ventilation.

8. Système de ventilateur selon la revendication 7, le dispositif de commande étant configuré pour générer un quatrième signal de commande pour une quatrième période de temps à la fin du temps d'expiration du cycle de ventilation, la pompe rotative étant configurée pour modifier la pression du gaz d'entraînement à une quatrième valeur sur la base du quatrième signal de commande reçu en provenance du dispositif de commande, et la quatrième valeur étant supérieure à la troisième valeur et inférieure à la première valeur.
